# FASCICULE DE BREVET EUROPEEN

(11) **EP 4 019 955 B1**
(45) Date de publication et mention de la délivrance du brevet: **05.02.2025**
(21) Numéro de dépôt: 21216017.0
(22) Date de dépôt: 20.12.2021
(51) Int. Cl.: G01N 27/327, A61B 5/145, A61B 5/1486

(54) **ÉLECTRODE POUR BIOCAPTEUR ENZYMATIQUE À MATÉRIAU FIBREUX, SON PROCÉDÉ DE PRÉPARATION ET LEDIT BIOCAPTEUR**
ELEKTRODE FÜR ENZYMATISCHEN BIOSENSOR MIT FASERIGEM MATERIAL, IHR HERSTELLUNGSVERFAHREN UND ENTSPRECHENDER BIOSENSOR
ELECTRODE FOR ENZYMATIC BIOSENSOR MADE FROM FIBROUS MATERIAL, METHOD FOR PREPARING SAME AND SAID BIOSENSOR

(30) Priorité: 22.12.2020 FR 2013874
(43) Date de publication de la demande: 29.06.2022
(73) Titulaire: Commissariat à l'Energie Atomique et aux Energies Alternatives, 75015 Paris (FR)
(72) Inventeur: REVOL-CAVALIER, Frédéric, 38054 GRENOBLE CEDEX 9 (FR); MAILLEY, Pascal, 38054 GRENOBLE CEDEX 9 (FR); PERRAULT, Natalie, 38054 GRENOBLE CEDEX 9 (FR); PERWUELZ, Anne, 59700 MARCQ EN BAROEUL (FR); VROMAN, Philippe, 59118 WAMBRECHIES (FR)
(74) Mandataire: Brevalex

(56) Documents cités:
- EP-A1- 3 385 708
- WO-A1-87/06701
- JP-A- H04 109 158
- US-A- 4 337 138

## Description

### DOMAINE TECHNIQUE

La présente invention appartient au domaine technique des dispositifs électrochimiques et notamment des biocapteurs ou bioréacteurs enzymatiques à détection électrochimique.

Plus particulièrement, la présente invention concerne une électrode pour biocapteur enzymatique se présentant sous forme d'un matériau fibreux comprenant notamment des fibres de matières différentes dont certaines sont adaptées pour assurer le rôle de conduction électrique et d'autres sont adaptées pour la fixation de enzymes permettant la transformation d'un analyte d'intérêt à doser en un produit réactionnel mesurable.

La présente invention concerne également un procédé de préparation d'une telle électrode et ses utilisations.

### ÉTAT DE LA TECHNIQUE ANTÉRIEURE

Un biocapteur enzymatique ou un bioréacteur enzymatique permet de transformer un analyte biologique ou chimique par l'intermédiaire d'une ou plusieurs enzyme(s). Dans le cas d'un biocapteur enzymatique, le produit de réaction d'un analyte par une enzyme ou une cascade enzymatique est mesuré, permettant de remonter à l'analyte initial non détectable. Le capteur de glucose est l'exemple de capteur enzymatique le plus connu, il permet le suivi des personnes diabétiques. La détection du produit de la réaction enzymatique peut être optique (colorimétrie ou fluorescence), elle peut également être électrochimique si le produit peut échanger des électrons avec un matériau conducteur.

Quel que soit le type de biocapteur considéré, il comprend 2 éléments distincts que sont 1) une ou plusieurs enzymes permettant de transformer l'analyte à doser (ici le glucose) en un produit réactionnel mesurable par une réaction électrochimique et 2) une électrode conductrice permettant la réaction électrochimique ou/et le transfert d'électrons vers une électronique de mesure.

Il existe 3 grands types de biocapteurs enzymatiques à détection électrochimique.

Le premier de ces types de biocapteurs enzymatiques ou « biocapteurs de première génération » utilise une enzyme produisant un produit qui peut être réduit ou oxydé directement sur l'électrode. A titre d'exemple, un tel biocapteur met en oeuvre la réduction ou l'oxydation du peroxyde d'hydrogène (H₂O₂) produit, à partir du glucose, par une réaction enzymatique, sur une électrode en présence d'oxygène.

Le deuxième type de biocapteurs enzymatiques dits « biocapteurs de deuxième génération » utilise un médiateur redox qui échange des électrons en solution avec le produit de réaction enzymatique. Le médiateur une fois modifié échange à son tour des électrons avec l'électrode conductrice du biocapteur. Ce type de biocapteur permet soit de s'affranchir de l'apport d'oxygène nécessaire au fonctionnement des bioréacteurs de première génération si le médiateur interagit directement avec le centre actif de l'enzyme soit d'abaisser le potentiel redox via l'utilisation d'un médiateur dit « rapide » (la cinétique du transfert d'électron avec la solution étant rapide) servant de navette redox entre l'électrode et l'un des produits de la réaction enzymatique tel que le H₂O₂ qui présente une vitesse de transfert électronique lente avec les électrodes.

Par ailleurs, les biocapteurs de première génération et de deuxième génération comprennent une électrode métallique recouverte par un biofilm contenant des enzymes emprisonnées dans un matériau poreux ou diffusionnel laissant passer l'analyte à doser. Ce dernier doit pénétrer dans le biofilm pour être mis en contact avec les enzymes. Une fois la réaction enzymatique effective, le produit de la réaction doit arriver par diffusion vers l'électrode pour être détecté. Cette diffusion réduit l'efficacité du capteur car tous les produits de la réaction ne diffusent pas vers l'électrode. Pour augmenter statistiquement le contact entre le produit de la réaction et l'électrode, il est possible de réduire l'épaisseur de la couche contenant les enzymes. Dans ce cas on limite la quantité d'enzymes disponibles et on réduit la plage d'utilisation et/ou la sensibilité du capteur. En effet, le substrat est rapidement en excès par rapport aux enzymes disponibles et le capteur est rapidement saturé. De plus, la sensibilité du capteur est fixée par la quantité d'enzymes actives immobilisées à l'électrode.

Les biocapteurs de troisième génération utilisent des enzymes directement couplées à l'électrode conductrice et ayant la capacité de directement échanger des électrons avec elle. Pour réaliser ces électrodes, plusieurs matériaux ont été utilisés avec des technologies de mise en oeuvre diverses. Par exemple, on peut citer des électrodes à base de billes nanométriques métalliques et notamment en or, en platine ou en carbone ; de nanofils d'oxyde de zinc (ZnO) sur électrode d'argent et voire de nanofils réalisés par électrospinning. Une liste de matériaux est donnée par Mahbur Rahman *et al,* 2010 [1]. Les électrodes des biocapteurs de troisième génération sont difficiles à réaliser et demandent des technologies coûteuses à mettre en place ce qui les rend peu utilisées. Par ailleurs, la chimie de fixation des enzymes est dictée par la nature de l'électrode mise en oeuvre.

Un avantage de l'utilisation des électrodes tridimensionnelles réside dans le fait que l'échantillon liquide susceptible de contenir l'analyte à doser est partiellement contenu dans l'électrode contrairement à une électrode planaire pour laquelle l'électrode baigne dans la solution. De fait cet avantage permet d'effectuer des réactions quantitatives (notion de bioréacteur) de la totalité du substrat contenu dans l'électrode ce qui n'est pas possible ou au demeurant très long avec une électrode planaire. Cette fonctionnalité est intéressante dans le cas d'une bioproduction électroenzymatique d'un produit chimique mais aussi dans le cadre des biocapteurs pour une mesure intégrative de la quantité de substrat permettant une meilleure sensibilité de la détection.

Un autre inconvénient de ces électrodes tridimensionnelles est leur caractère rigide. En effet, souvent à base de métal, l'électrode est rigide et ne se déforme pas. Le volume d'échantillon liquide susceptible de contenir l'analyte à doser qui « rentre » dans l'électrode tridimensionnelle a une valeur déterminée par le volume libre entre les nanostructures du type nanofils ou nanobilles, constitutifs de l'électrode. Ce liquide est non seulement difficilement agitable mais aussi difficilement expulsable.

WO87/06701 A1, EP3385708 A1, US4337138 A et JPH04109158 A divulguent des électrodes se présentant sous forme d'un matériau fibreux.

Les inventeurs se sont donc fixé pour but de proposer un biocapteur enzymatique et notamment une électrode pour biocapteur enzymatique dont le procédé de préparation est facile et donc peu coûteux et dans laquelle l'échantillon liquide susceptible de contenir l'analyte à doser peut être présent en une quantité modulable et peut être facilement agitable et expulsable.

### EXPOSÉ DE L'INVENTION

La présente invention permet d'atteindre le but que se sont fixé les inventeurs et de résoudre tout ou partie des problèmes techniques des biocapteurs enzymatiques de l'art antérieur.

Pour ce faire, les inventeurs proposent une électrode pour biocapteur enzymatique du type électrode tridimensionnelle qui présente donc les avantages d'un tel type à savoir une électrode apte à contenir l'échantillon liquide susceptible d'inclure l'analyte à doser.

La particularité de l'électrode selon l'invention est qu'elle se présente sous forme d'un matériau fibreux, compressible et résilient, ce qui lui permet de s'adapter à la quantité d'échantillon liquide à mesurer. Cette adaptation a un double avantage. Elle permet de mesurer une quantité d'échantillon liquide modulable dans une fourchette correspondant à sa capacité de se déformer. Elle permet également par simple pression d'agiter l'échantillon liquide contenu dans la structure pour faciliter la rencontre entre les enzymes et l'analyte en appliquant alternativement une pression positive et négative sur l'électrode. Enfin l'échantillon liquide contenu dans l'électrode tridimensionnelle peut être expulsé, par pression, à la fin de la mesure. La compressibilité et la résilience de l'électrode permettent de remplir et vider l'électrode régulièrement au début et à la fin de la mesure. Cette capacité permet de réaliser des mesures séquentielles (remplissage et expulsion du liquide avant une autre mesure)

On entend par « matériau compressible », un matériau fibreux poreux présentant la capacité à augmenter ou réduire son volume lorsqu'il absorbe ou expulse du liquide par compression ou extension mécanique ou par capillarité. La déformation de l'électrode selon l'invention est comprise entre 5% et 200% et plus particulièrement entre 30% et 90%. Cette déformation est soit directement obtenue à l'aide par exemple d'un banc de traction, soit calculée à l'aide d'un mesureur d'épaisseur, d'un pied à coulisse, d'une observation au microscope, d'un banc de traction ou de tout autre appareil permettant de donner la distance entre 2 points ou 2 surfaces tout en permettant d'appliquer une pression donnée sur la zone où est effectuée la mesure. Dans les deux cas la valeur de la compression est obtenue à partir de la différence d'épaisseur du matériau, sec ou mouillé, entre une première épaisseur obtenue sans application d'une pression ou sous l'application d'une pression inférieure ou égale à 1 kPa, et une deuxième épaisseur obtenue sous l'application d'une pression supérieure à 1 kPa et notamment une pression comprise entre 1 kPa (borne non incluse) et 50 kPa. Par « échantillon mouillé », on entend un échantillon mis dans une solution aqueuse pendant au moins 1 nuit afin qu'il soit totalement imprégné, l'échantillon est ensuite placé sur l'instrument de mesure sans être séché ou égoutté. La mesure sera typiquement faite à l'aide d'un banc de traction permettant de mesurer un déplacement et ainsi une déformation en fonction d'une force (F) appliquée sur un échantillon de dimension définie (S) revenant donc à une valeur de pression (P=F/S), pression allant de 100 Pa à 50 kPa, sans précharge et à une vitesse de test moyenne de 10 mm/min.

On entend par « résilience », la capacité du matériau à retrouver sa forme initiale lorsqu'aucune force mécanique n'est appliquée.

Plus particulièrement, la présente invention concerne une électrode pour biocapteur enzymatique selon l'énoncé de la revendication indépendante 1. Plusieurs modes de réalisation différents sont envisageables pour l'électrode selon l'invention.

Dans un premier exemple de réalisation qui ne fait pas partie de l'invention, l'électrode comprend ou est constituée de fibres conductrices de l'électricité, tout ou partie desquelles sont fonctionnalisées par des enzymes, identiques ou différentes.

Dans ce premier exemple, l'électrode comprend ou n'est constituée que de fibres conductrices de l'électricité sur tout ou partie desquelles sont fixées, de façon chimique, des enzymes, identiques ou différentes.

Dans un deuxième exemple de réalisation qui ne fait pas partie de l'invention, l'électrode comprend ou est constituée de fibres conductrices de l'électricité, tout ou partie desquelles sont fonctionnalisées par des enzymes, identiques ou différentes, et de fibres non-conductrices de l'électricité.

Dans ce deuxième exemple, l'électrode ne comprend ou n'est constituée que de deux types de fibres avec 1) des fibres conductrices de l'électricité sur tout ou partie desquelles sont fixées, de façon chimique, des enzymes, identiques ou différentes et 2) des fibres non-conductrices de l'électricité. Lorsque seule une partie des fibres conductrices sont fonctionnalisées par des enzymes, les fibres non-conductrices de l'électricité et éventuellement les fibres conductrices de l'électricité non fonctionnalisées jouent le rôle de fibres résilientes pour accentuer le caractère résilient du matériau fibreux constituant l'électrode.

Dans un troisième mode de réalisation, l'électrode selon l'invention comprend ou est constituée de fibres conductrices de l'électricité et de fibres non-conductrices de l'électricité, tout ou partie desquelles sont fonctionnalisées par des enzymes, identiques ou différentes.

Dans ce troisième mode, l'électrode comprend ou n'est constituée que de deux types de fibres avec 1) des fibres conductrices de l'électricité et 2) des fibres non-conductrices de l'électricité sur tout ou partie desquelles sont fixées, de façon chimique, des enzymes, identiques ou différentes. Lorsque seule une partie des fibres non-conductrices sont fonctionnalisées par des enzymes, les autres fibres non-conductrices et non fonctionnalisées jouent le rôle de fibres résilientes pour accentuer le caractère résilient du matériau fibreux constituant l'électrode selon l'invention.

Dans un quatrième mode de réalisation, l'électrode selon l'invention comprend ou est constituée de fibres conductrices de l'électricité, d'un premier type de fibres non-conductrices de l'électricité, non fonctionnalisées par des enzymes et d'au moins un second type de fibres non-conductrices de l'électricité, différent du premier type de fibres non-conductrices de l'électricité, tout ou partie desquelles sont fonctionnalisées par des enzymes, identiques ou différentes.

Ce quatrième mode de réalisation est une forme de mise en oeuvre particulière du troisième mode de réalisation.

Dans ce quatrième mode, l'électrode comprend ou est constituée d'au moins trois types de fibres avec 1) des fibres conductrices de l'électricité, 2) un premier type de fibres non-conductrices de l'électricité, non fonctionnalisées par des enzymes et 3) au moins un second type de fibres non-conductrices de l'électricité différent du premier type, sur tout ou partie desquelles sont fixées, de façon chimique, des enzymes, identiques ou différentes. Les fibres non-conductrices de l'électricité du premier type et éventuellement les fibres non-conductrices de l'électricité du au moins un second type et non fonctionnalisées par des enzymes jouent le rôle de fibres résilientes.

L'expression « au moins un second type de fibres non-conductrices de l'électricité » signifie que, dans ce quatrième mode de réalisation, il est possible d'avoir un second type de fibres non-conductrices de l'électricité, différent du premier type mais aussi deux, trois, quatre ou encore cinq types différents de fibres non-conductrices de l'électricité, dont tout ou partie sont fonctionnalisées par des enzymes, ces différents types de fibres étant non seulement différents du premier type de fibres non-conductrices de l'électricité mais aussi différents les uns des autres. Les différents types de fibres non-conductrices de l'électricité et notamment le premier type et le second type de fibres non-conductrices de l'électricité, mis en oeuvre dans ce mode de réalisation se distinguent, les uns des autres et notamment l'un de l'autre, au niveau de leur composition chimique et/ou de leur titrage.

Dans ce quatrième mode de réalisation, il est possible d'avoir différents types de fibres non-conductrices de l'électricité avec, pour chaque type, au moins une partie des fibres fonctionnalisées par une enzyme spécifique. A cet effet, chaque type de fibres peut porter une fonction chimique adaptée à la fixation d'une enzyme spécifique. Ce mode de réalisation permet d'obtenir une électrode comprenant plusieurs enzymes disposées sur des fibres différentes et ainsi de réaliser une électrode complexe composée de fibres mélangées spécifiquement couplées à des enzymes différentes pour, par exemple, enchaîner des réactions enzymatiques en cascade. A noter, par ailleurs, que, comme, dans tous les modes de réalisation d'une électrode selon l'invention, il est envisagé de fonctionnaliser les fibres mises en oeuvre par des enzymes différentes, l'utilisation de ces électrodes pour réaliser des réactions enzymatiques en cascade est possible.

Quel que soit le mode de réalisation envisagé, l'électrode selon l'invention se présente sous forme d'un matériau fibreux, compressible et résilient.

Le troisième mode et le quatrième mode de réalisation de l'électrode selon l'invention offrent l'avantage de différencier les fibres conductrices de l'électricité des fibres portant les enzymes. En effet, il est possible de constituer l'électrode tridimensionnelle selon l'invention avec des fibres de différentes natures permettant de localiser les fibres porteuses d'enzymes par rapport aux fibres conductrices.

Cet avantage est double : il permet de s'assurer que les fibres conductrices de l'électricité ne sont pas porteuses de fonctions chimiques qui pourraient modifier leur conductivité ou leur capacité de transfert électronique entre les produits de la réaction enzymatique et leur réaction électrochimique sur les fibres conductrices. Cette séparation entre les fibres porteuses d'enzymes et les fibres conductrices de l'électricité permet également de choisir la nature des fibres sur lesquelles sont fixées les enzymes, ce qui permet de choisir des réactions chimiques plus simples ou avec un rendement chimique plus élevé. La chimie de fixation des enzymes n'est pas dictée par la nature de l'électrode mise en oeuvre.

Enfin un autre avantage de l'électrode selon l'invention, à savoir selon le troisième mode et le quatrième mode de réalisation, est que l'on peut modifier à volonté le ratio de fibres conductrices et de fibres porteuses de l'enzyme pour réaliser des bioréacteurs différents avec les mêmes fibres, seul leur ratio étant différent ce qui permet de s'adapter à la concentration des milieux à tester.

Dans le troisième mode et le quatrième mode de réalisation de l'électrode selon l'invention tels que définis ci-dessus, les ratios de fibres mises en oeuvre sont de 5% à 90% de fibres conductrices de l'électricité, de 10% à 80% de fibres non-conductrices de l'électricité et porteuses d'enzymes et de 0% à 80% de fibres résilientes. Plus particulièrement, ces ratios sont de 50% à 70% de fibres conductrices de l'électricité, de 10% à 50% de fibres non-conductrices de l'électricité et porteuses d'enzymes et de 0% à 30% de fibres résilientes.

De plus, dans le troisième mode et le quatrième mode de réalisation de l'électrode selon l'invention tels que définis ci-dessus, la distance moyenne entre une fibre conductrice de l'électricité et une fibre porteuse d'enzymes est de 100 nm à 200 µm.

L'électrode selon l'invention se présente sous forme d'un matériau fibreux i.e. un matériau dont les éléments constitutifs essentiels sont des fibres. Ce matériau peut se présenter sous forme d'un textile tissé, sous forme d'un textile tricoté ou sous forme d'un textile non tissé.

Par « fibre », on entend, dans le cadre de la présente invention, une structure unidimensionnelle ou sensiblement unidimensionnelle présentant une épaisseur ou un diamètre variant de 500 nm à 100 µm, notamment, de 1 µm à 50 µm et, en particulier, entre 4 µm et 30 µm.

La longueur des fibres mises en oeuvre dans la présente invention est choisie en fonction de la technique utilisée pour préparer le matériau fibreux de l'électrode. Typiquement, la longueur des fibres mises en oeuvre est supérieure à 4 mm. Cette longueur peut être comprise entre 5 mm et 120 mm et, en particulier, entre 6 mm et 20 mm et ce, notamment lorsque ces fibres sont utilisées pour préparer des textiles non tissés via le procédé airlaid. En variante, la longueur des fibres mises en oeuvre peut être comprise entre 15 mm et 180 mm et, en particulier, entre 30 mm et 120 mm et ce, notamment lorsque ces fibres sont utilisées pour préparer des textiles non tissés cardés. En variante encore, les fibres mises en oeuvre dans la présente invention peuvent présenter une longueur supérieure à 180 mm et, en particulier, supérieure à 200 mm et ce, notamment lorsque ces fibres sont utilisées pour préparer des textiles tissés ou tricotés. Dans cette variante, les fibres peuvent se présenter sous forme de fils ou filaments et avoir été obtenus par traitement de fibres plus courtes comme, par exemple, une filature.

Avantageusement, les fibres mises en oeuvre dans la présente invention ont un titrage compris entre 0,2 dTex et 30 dTex et notamment entre 0,3 dTex et 20 dTex.

Dans le cadre de la présente invention, on entend par « fibre conductrice de l'électricité » une fibre telle que précédemment définie, conductrice de l'électricité par nature ou suite à un traitement. Une telle fibre est choisie dans le groupe constitué par les fibres métalliques, les fibres en carbone, les fibres en polymères ou copolymères conducteurs de l'électricité, les fibres en composites polymères chargés (CPC) et les fibres rendues conductrices via enduction ou métallisation.

A titre d'exemples de fibres métalliques, on peut citer des fibres en métal, en oxyde métallique, en nitrure métallique et en sulfure métallique, dans lesquels le métal est choisi dans le groupe constitué par l'or, le cuivre, l'inox, l'argent, le nickel, l'aluminium, le platine, le palladium, le molybdène ou un de leurs alliages.

Les fibres en carbone utilisables dans le cadre de la présente invention peuvent appartenir à l'une quelconque des trois principales familles de fibres en carbone que sont les fibres ex-cellulose obtenues par carbonisation de matériaux tels que le papier ou la viscose ; les fibres ex-PAN produites en utilisant, comme précurseur, du polyacrylonitrile (PAN) ; et les fibres ex-brais fabriquées à partir de résidus aromatiques de la distillation du pétrole ou du charbon. A titre d'exemples particuliers de fibres en polymères ou copolymères conducteurs de l'électricité, on peut citer des fibres en polyaniline (PANI), en poly(3,4-éthylènedioxythiophène) couplé au poly(styrène sulfonate) de sodium (PEDOT:PSS), en polypyrrole ou en polyacétylène. A noter que ces polymères ou copolymères conducteurs de l'électricité sont également désignés par l'expression « polymères intrinsèquement conducteurs (ICP) ».

Les fibres en composites polymères chargés (CPC) sont des fibres en polymères ou copolymères contenant des particules telles que des nanotubes de carbone, du noir de carbone, des particules métalliques ou des particules en polymères intrinsèquement conducteurs (ICP) comme ceux précédemment cités, ou des mélanges de ces différents types de particules.

Les fibres conductrices de l'électricité utilisables dans le cadre de la présente invention regroupent aussi les fibres rendues conductrices par enduction via par exemple le dépôt d'un mélange de polymères ou copolymères avec des particules conductrices, ou par métallisation utilisant par exemple les technologies d'électrodéposition, de plasma ou de CVD (pour « Chemical Vapor Déposition »). A titre d'exemple plus particulier de fibres rendues conductrices par enduction, on peut citer des fibres enduites d'or.

Dans un mode de réalisation particulier, les fibres conductrices de l'électricité mises en oeuvre dans le cadre de la présente invention sont des fibres en carbone et, plus particulièrement, des fibres ex-PAN.

Dans le cadre de la présente invention, on entend par « fibre nonconductrice de l'électricité » une fibre telle que précédemment définie choisie parmi les fibres naturelles non-conductrices de l'électricité et les fibres chimiques non-conductrices de l'électricité.

A titre d'exemples particuliers de fibres naturelles non-conductrices de l'électricité, on peut citer les fibres comprenant ou en un matériau choisi dans le groupe constitué par le coton, la laine, le lin, le jute, la cellulose, le chanvre, le raphia, le sisal, la soie, le tussah, le byssus, les alginates, les polysaccharides et un de leurs mélanges.

Les fibres chimiques comprennent les fibres synthétiques et les fibres artificielles. A titre d'exemples particuliers de fibres chimiques non-conductrices de l'électricité, on peut citer les fibres comprenant ou en un matériau choisi dans le groupe constitué par le verre, la rayonne, le chitosan, les polyoléfines comme le polyéthylène (PE) ou le polypropylène (PP) ; le polytétrafluoroéthylène (PTFE) ; les polyesters comme le polyéthylène téréphtalate (PET) et le polyéthylène naphtalate (PEN) ; les polyamides ; les polyimides ; les polycarbonates (PC) et un de leurs mélanges.

Dans un mode de réalisation particulier, les fibres non-conductrices de l'électricité mises en oeuvre dans le cadre de la présente invention comprennent des fibres à bas point de fusion i.e. des fibres dont le point de fusion est inférieur à 190°C et notamment compris entre 105°C et 170°C. De telles fibres à bas point de fusion sont utilisables comme fibres sur lesquelles sont fixées, de façon covalente, les enzymes et/ou comme fibres résilientes. Un exemple particulier de telles fibres à bas point de fusion correspond aux fibres PET/coPET commercialisées par Fiber Partner^{®}. le troisième et le quatrième mode de réalisation de l'électrode selon l'invention tels que définis ci-dessus, le ratio de fibres non-conductrices de l'électricité à bas point de fusion est typiquement de 0 à 50% et notamment de 10% à 30%. L'utilisation de fibres à bas point de fusion est intéressante car elle permet de lier les fibres entres elles par un traitement thermiques adapté permettant la fonte partielle de ces fibres.

Les enzymes mises en oeuvre dans l'électrode selon la présente invention sont notamment choisies dans le groupe constitué par les oxydoréductases, les oxygénases, les peroxydases, les catalases, les transhydrogénases, les déshydrogénases, les transférases, les hydrolases, les lyases et les ligases. Avantageusement, les enzymes mises en oeuvre dans l'électrode selon la présente invention sont des oxydoréductases.

Plus particulièrement, les enzymes mises en oeuvre dans l'électrode selon la présente invention sont choisies dans le groupe constitué par les créatinases, les créatine amidohydrolases, les réductases, les estérases, les catalases, les urate oxydases, les galactose oxydases, les histamine oxydases, les choline oxydases, les glucose oxydases, les glucose déshydrogénases, les fructose déshydrogénases, les gluconate déshydrogénases, les glutamate oxydases, les glutamate déshydrogénases, les cholestérol oxydases, les cholestérol estérases, les lactate oxydases, les lactate déshydrogénases, les ascorbate oxydases, les pyruvate oxydases, les alcool oxydases, les alcool déshydrogénases, les aldéhyde déshydrogénases, les bilirubine oxydases, les choline oxydases, les xanthine oxydases, les aminoacide oxydases, les peroxydases, les uréases, les formate déshydrogénases, les pyruvate déshydrogénases, les malate déshydrogénases, les méthylamine déshydrogénases, les succinate déshydrogénases, les fumarate réductases, les p-crésolméthylhydroxylases, les glutamate oxaloacétate transaminases et les glutamate pyruvate transaminases.

Dans le cadre de l'électrode selon la présente invention, tout ou partie des fibres sont fonctionnalisées par des enzymes, identiques ou différentes, telles que précédemment définies. Cette fonctionnalisation met en oeuvre au moins une liaison chimique. Par « liaison chimique », on entend non seulement une liaison chimique covalente mais aussi une liaison chimique non-covalente telle qu'une liaison ionique, une liaison hydrogène, une liaison hydrophobe ou une liaison de van der Waals. Ainsi, selon l'invention, les enzymes sont fixées, greffées ou immobilisées, de façon covalente ou non-covalente, sur la partie latérale des fibres non-conductrices de l'électricité. Cette fixation, ce greffage ou cette immobilisation peut être localisé(e) sur des zones limitées et définies de cette surface ou, au contraire, selon l'invention, les enzymes sont réparties sur l'ensemble de cette surface.

Dans le cadre de la présente invention, la fixation, le greffage ou l'immobilisation des enzymes sur les fibres non-conductrices de l'électricité peut être directe ou indirecte i.e. la fonctionnalisation des fibres non-conductrices de l'électricité par des enzymes est directe ou indirecte.

Lorsqu'elle est directe, la liaison chimique mise en oeuvre implique un atome présent à la surface de la fibre et un atome de l'enzyme.

Lorsque la fixation est indirecte, la fixation, le greffage ou l'immobilisation fait intervenir un bras espaceur (ou agent de jonction) lié, d'une part, à la surface de la fibre et, d'autre part, à une enzyme. Les liaisons mises en oeuvre peuvent être des liaisons covalentes ou non-covalentes. Le bras espaceur compile deux fonctionnalités, l'une consiste à donner de la mobilité à l'édifice (chimie carbone de type polyéthylène glycol (PEG), groupement alkyle, polytéréphtalate d'éthylène (PET), etc.), l'autre consiste à accrocher à l'électrode l'enzyme. Cette fonction d'accrochage peut être symétrique (la même à chaque bout du bras espaceur) ou asymétrique (par exemple, une fonction silane, thiol, aldéhyde, époxy...).

Afin de faciliter la liaison chimique entre l'enzyme et la surface d'une fibre dans l'électrode selon l'invention, l'enzyme et la surface de la fibre portent ou sont substituées, toutes deux, par au moins une fonction réactive, identique ou différente. Lorsqu'un bras espaceur est mis en oeuvre, ce dernier porte deux fonctions réactives, identiques ou différentes.

Par « fonction réactive », on entend, dans le cadre de la présente invention, une fonction choisie parmi une fonction carboxyle (susceptible de réagir avec une fonction amine ou alcool), un groupe aryle (tel que le pyrène, le naphtalène ou les polyaromatiques), une entité radicalaire, une fonction hydroxyle ou une fonction alcool (susceptible de réagir avec une fonction carboxyle ou isocyanate), une fonction amine (susceptible de réagir avec une fonction ester ou acide carboxylique), une fonction ester (susceptible de réagir avec une fonction amine), une fonction aldéhyde (susceptible de réagir avec une fonction hydrazide), une fonction hydrazide (susceptible de réagir avec une fonction aldéhyde), une fonction cétone (susceptible de réagir avec deux fonctions alcool en vue d'une acétalisation), une fonction époxy (susceptible de réagir avec une fonction amine), une fonction isocyanate (susceptible de réagir avec une fonction hydroxyle), une fonction maléimide (susceptible de réagir avec une fonction thiol, une fonction amine ou une fonction diène), une fonction diène (susceptible de réagir avec une fonction maléimide), une fonction thiol (susceptible de réagir avec une fonction maléimide ou une autre fonction thiol), une fonction phosphonate (susceptible de chélater des ions zirconium (Zr⁴⁺) ou titane (Ti⁴⁺)), une fonction chélatant des ions zirconium (Zr⁴⁺), des ions titane (Ti⁴⁺), des ions fer (Fe³⁺) et/ou des ions gallium (Ga³⁺) (susceptible d'immobiliser des phosphopeptides), une biotine (susceptible de se lier à une avidine ou une streptavidine), une avidine ou une streptavidine (susceptible de se lier à une biotine) et une étiquette polyhistidine (susceptible de se lier à des ions métalliques comme des ions nickel (Ni²⁺) ou des ions cobalt (Co²⁺)).

Cette ou ces fonction(s) réactive(s) peu(ven)t être naturellement présente(s) au niveau de l'enzyme, de la surface de la fibre ou du bras espaceur. Par exemple, une enzyme comprenant, dans sa séquence en acides aminés, au moins une cystéine présente naturellement une fonction thiol. De même, les bras espaceurs portent naturellement deux fonctions réactives, identiques ou différentes.

En variante, cette fonction réactive peut devoir être introduite au niveau de l'enzyme et/ou de la surface de la fibre.

En ce qui concerne l'enzyme, une fonction réactive peut être introduite, par fonctionnalisation, de l'acide aminé en position C-terminale, de l'acide aminé en position N-terminale et/ou de chaînes latérales d'acides aminés dans la séquence d'acides aminés. Toute technique de fonctionnalisation connue de l'homme du métier est utilisable à cet effet. Par exemple, en vue d'introduire une fonction thiol dans une enzyme, il est possible d'utiliser un réactif du type isocyanate, isothiocyanate ou ester de succinate. Il est évident que l'introduction d'une fonction réactive dans une enzyme ne doit pas substantiellement modifier l'activité enzymatique de cette dernière.

En ce qui concerne la surface de la fibre, il est possible d'introduire une fonction réactive par toute technique de fonctionnalisation d'une surface métallique, d'une surface en carbone ou d'une surface polymère connue de l'homme du métier et notamment via un traitement oxydant. En effet, un traitement oxydant vise à oxyder la surface de la fibre en fixant et/ou en introduisant, sur cette dernière, des groupements, identiques ou différents, riches en oxygène i.e. des groupements, identiques ou différents, comprenant au moins un atome d'oxygène et notamment choisi dans le groupe constitué par un groupement carboxylique (-C(=O)OH), un groupement hydroxyle (-OH), un groupement alcoxyle (-OX avec X représentant un groupe alkyle, un groupe acyle ou un groupe aryle), un groupement carbonyle (-C(=O)-), un groupement percarbonique (-C(=O)-O-OH) et un groupement amide (-C(=O)NH₂).

Un tel traitement oxydant repose sur deux grands types de modifications de surface fondés sur :
- des traitements physiques tels qu'un traitement par plasma notamment d'oxygène, un traitement aux UV, un traitement aux rayons X ou gamma, un traitement par irradiation aux électrons et aux ions lourds ;
- des traitements chimiques tels qu'un traitement à la potasse alcoolique, un traitement avec un mélange d'acide sulfurique (H₂SO₄) et de peroxyde d'hydrogène (H₂O₂) également connu sous l'appellation « mélange piranha », un traitement par un acide fort (HCl, H₂SO₄, HNO₃, HClO₄), un traitement à la soude, un traitement par un oxydant fort (KMnO₄, K₂Cr₂O₇, KClO₃ ou CrO₃ dans l'acide chlorhydrique, l'acide sulfurique ou l'acide nitrique), un traitement à l'ozone et un traitement thermique sous atmosphère oxygénée (O₂, H₂O,...).

La présente invention concerne un procédé de préparation d'une électrode pour biocapteur enzymatique telle que précédemment définie, ce procédé étant énoncé dans les revendications indépendantes 8, 9 ou 10 sous la forme de trois alternatives.

Le procédé pour préparer une électrode selon le troisième mode et le quatrième mode de réalisation tels que précédemment définis comprend au moins les deux étapes suivantes:
- le mélange de fibres conductrices de l'électricité avec des fibres non-conductrices de l'électricité et
- la fonctionnalisation de certaines des fibres non-conductrices de l'électricité avec des enzymes,
ces deux étapes étant réalisées l'une après l'autre dans n'importe quel ordre.

Dans une première variante, ce procédé selon l'invention comprend les étapes telles que définies dans la revendication indépendante 8, à savoir :
a1) la fonctionnalisation des fibres non-conductrices de l'électricité par des enzymes, sur l'ensemble de la partie latérale desdites fibres non-conductrices de l'électricité ;
b1) le mélange des fibres non-conductrices de l'électricité fonctionnalisées par des enzymes avec des fibres conductrices de l'électricité non fonctionnalisées par des enzymes et éventuellement des fibres non-conductrices de l'électricité et
c1) la mise en forme du mélange de fibres obtenues à l'étape b1) de façon à obtenir un matériau fibreux.

Préalablement à l'étape a1), il est possible de soumettre les fibres conductrices ou non-conductrices de l'électricité à un traitement oxydant tel que précédemment décrit.

Par « mélange », on entend un mélange de différents types de fibres conductrices de l'électricité, un mélange de différents types de fibres non-conductrices de l'électricité mais aussi un mélange d'au moins un type de fibres conductrices de l'électricité et d'au moins un type de fibres non-conductrices de l'électricité.

Dans une deuxième variante, ce procédé selon l'invention comprend les étapes telles que définies dans la revendication indépendante 9, à savoir :
a2) la fonctionnalisation des fibres non-conductrices de l'électricité par des fonctions réactives sur l'ensemble de la partie latérale desdites fibres non-conductrices de l'électricité ;
b2) le mélange des fibres non-conductrices de l'électricité fonctionnalisées par des fonctions réactives avec des fibres conductrices de l'électricité non fonctionnalisées par des enzymes et éventuellement des fibres non-conductrices de l'électricité ; et
c2) la mise en forme du mélange de fibres obtenues à l'étape b2) de façon à obtenir un matériau fibreux ;
la fonctionnalisation par des enzymes des fibres non-conductrices de l'électricité préalablement fonctionnalisées par des fonctions réactives étant réalisée soit après l'étape b2) et préalablement à l'étape c2), soit après l'étape c2).

Cette deuxième variante présente deux alternatives selon que la fixation, le greffage ou l'immobilisation des enzymes sur les fibres non-conductrices de l'électricité fonctionnalisées par des fonctions réactives est réalisée sur le mélange de l'étape b2) (première alternative) ou une fois la mise en forme des fibres réalisée (deuxième alternative).

Lors de l'étape a2), la fonctionnalisation par des fonctions réactives des fibres non-conductrices de l'électricité consiste (i) à soumettre les fibres à un traitement oxydant, (ii) à fixer, greffer ou immobiliser, sur les fibres, des fonctions réactives telles que précédemment définies, et/ou (iii) à fixer, greffer ou immobiliser, sur les fibres, des bras espaceurs portant des fonctions réactives.

Dans une troisième variante, ce procédé selon l'invention comprend les étapes telles que définies dans la revendication indépendante 10, à savoir :
a3) le mélange de fibres conductrices de l'électricité et de fibres non-conductrices de l'électricité, et
b3) la mise en forme du mélange de fibres obtenues à l'étape a3) de façon à obtenir un matériau fibreux,
la fonctionnalisation par des enzymes des fibres non-conductrices de l'électricité sur l'ensemble de la partie latérale desdites fibres non-conductrices de l'électricité étant réalisée soit après l'étape a3) et préalablement à l'étape b3), soit après l'étape b3).

Cette troisième variante présente également deux alternatives selon que la fixation, le greffage ou l'immobilisation des enzymes sur les fibres conductrices ou sur les fibres non-conductrices de l'électricité est réalisé sur le mélange de l'étape a3) (première alternative) ou une fois la mise en forme des fibres réalisée (deuxième alternative).

L'homme du métier saura choisir, sans effort inventif, les fonctions réactives que doivent porter les enzymes et celles que doivent porter, de façon naturelle, les fibres non-conductrices de l'électricité pour obtenir une fixation, un greffage ou une immobilisation spécifique des enzymes sur ces seules fibres présentes dans le mélange de fibres mis en oeuvre.

Quelle que soit la variante de procédé envisagée, toute technique de mise en forme de fibres pour produire un matériau fibreux tissé ou non tissé est utilisable lors des étapes c1), c2) et b3).

A titre d'exemples de techniques utilisables lors des étapes c1), c2) et b3) du procédé selon l'invention pour produire un matériau fibreux non tissé, on peut citer les techniques par voie sèche, avec les procédés de cardage ou airlaid, soit les techniques par voie fondue avec les procédés spunbond, meltblown, électrospinning, soit encore les techniques par voie humide. Avantageusement, la mise en forme lors desdites étapes c1), c2) et b3) est réalisée en utilisant une technique par voie sèche comme le procédé de cardage ou le procédé airlaid.

A titre d'exemples de techniques utilisables lors des étapes c1), c2) et b3) du procédé selon l'invention pour produire un matériau fibreux tissé ou tricoté, on peut citer les techniques de tissage ou de tricotage.

Le matériau fibreux tissé, tricoté ou non tissé obtenu suite aux étapes c1), c2) et b3) présente une masse surfacique entre 100 g/m² et 800 g/m² et notamment entre 200 g/m² et 300 g/m².

Le matériau fibreux obtenu suite aux étapes c1), c2) et b3) peut être soumis à une consolidation. En d'autres termes, le procédé selon l'invention peut comprendre suite aux étapes c1), c2) et b3) une étape de consolidation. Toute technique de consolidation des matériaux fibreux non tissés, tissés ou tricotés est utilisable à cet effet. Ainsi, le matériau fibreux obtenu suite aux étapes c1), c2) et b3) et notamment le matériau fibreux non tissé obtenu suite aux étapes c1), c2) et b3) peut être consolidé par différents mode de liage, et notamment par aiguilletage, hydroliage, thermoliage, calandrage ou encore par liage chimique, stitchbonding ou une combinaison de ces différents modes de liage. Typiquement, le matériau fibreux obtenu suite aux étapes c1), c2) et b3) et notamment le matériau fibreux non tissé obtenu suite aux étapes c1), c2) et b3) peut être consolidé par liage mécanique suivi d'un liage thermique.

La présente invention concerne également un biocapteur enzymatique à détection électrochimique, comprenant une électrode telle que précédemment définie. Un tel biocapteur peut également être désigné sous l'expression de « bioréacteur enzymatique à détention électrochimique ».

Dans ce biocapteur, l'électrode objet de l'invention joue le rôle d'électrode de travail (WE) grâce aux fibres conductrices de l'électricité qu'elle contient. A titre de contre-électrode (CE), on peut utiliser une grille de platine, un fil de platine, une plaque de titane platiné, une pâte de noir de carbone et de carbone activé encapsulés dans une grille d'inox ou une pâte de noir de carbone, de carbone activé et de téflon encapsulés dans une grille d'inox. A titre d'électrode de référence (RE), on peut utiliser une électrode au calomel saturé comme une électrode au calomel saturé en chlorure de potassium ou en chlorure de sodium, une électrode Ag/AgCI ou encore un fil de platine.

Un tel biocapteur est notamment utile pour la détection et l'éventuelle quantification d'un analyte d'intérêt notamment choisi dans le groupe constitué par l'éthanol, le glucose, le microbiote dermique, des toxines, un composé chimique ou biologique associé à une condition médicale, des nutriments, des sous-produits métaboliques comme l'urée ou le cholestérol, des hormones, des ligands environnementaux ou l'une quelconque de leurs combinaisons. Une telle utilisation présente des applications dans le domaine médical, agro-alimentaire ou environnemental.

### BRÈVE DESCRIPTION DES DESSINS

La Figure 1 est une représentation schématisée du procédé de préparation d'une électrode telle que définie dans le quatrième mode de réalisation selon l'invention, ledit procédé étant tel que défini dans la première variante du procédé selon l'invention.
La Figure 2 est une représentation schématisée du procédé de préparation d'une électrode telle que définie dans le quatrième mode de réalisation selon l'invention, ledit procédé étant tel que défini dans la deuxième variante du procédé selon l'invention.
La Figure 3 est une représentation schématisée du procédé de préparation d'une électrode telle que définie dans le quatrième mode de réalisation selon l'invention, ledit procédé étant tel que défini dans la troisième variante du procédé selon l'invention.

### EXPOSÉ DÉTAILLÉ DE MODES DE RÉALISATION PARTICULIERS

### I. Electrode pour biocapteur enzymatique selon l'invention.

L'électrode selon l'invention est un matériau non tissé et comprend :
- des fibres conductrices de l'électricité que sont des fibres de carbone de diamètre entre 4 µm et 8 µm provenant typiquement de multifilament à base de Polyacrylonitrile (PAN),
- des fibres non-conductrices de l'électricité et porteuses d'enzymes que sont des fibres en PET de titre compris entre 0,3 dTex et 17 dTex et notamment entre 0,5 dTex et 2 dTex sur lesquelles sont fixées, de manière covalente, des enzymes du type oxydoréductases, glucose oxydases, et
- des fibres résilientes que sont des fibres bas point de fusion, comme par exemple des fibres LowMelt bicomposantes PET/coPET, ayant un titre allant de 2 dTex à 12 dTex.

Dans cette électrode, le ratio des fibres mises en oeuvre est entre 50% et 80% de fibres carbone, entre 10% et 50% de fibres porteuses d'enzymes, et entre 10% et 30% de fibres résilientes.

Les fibres sont entremêlées entre elles avec des zones de contact où les fibres conductrices de l'électricité sont en contact entre elles et avec les fibres porteuses des enzymes et d'autres zones ou les fibres sont éloignées les unes des autres.

### II. Procédé de préparation de l'électrode selon la première variante.

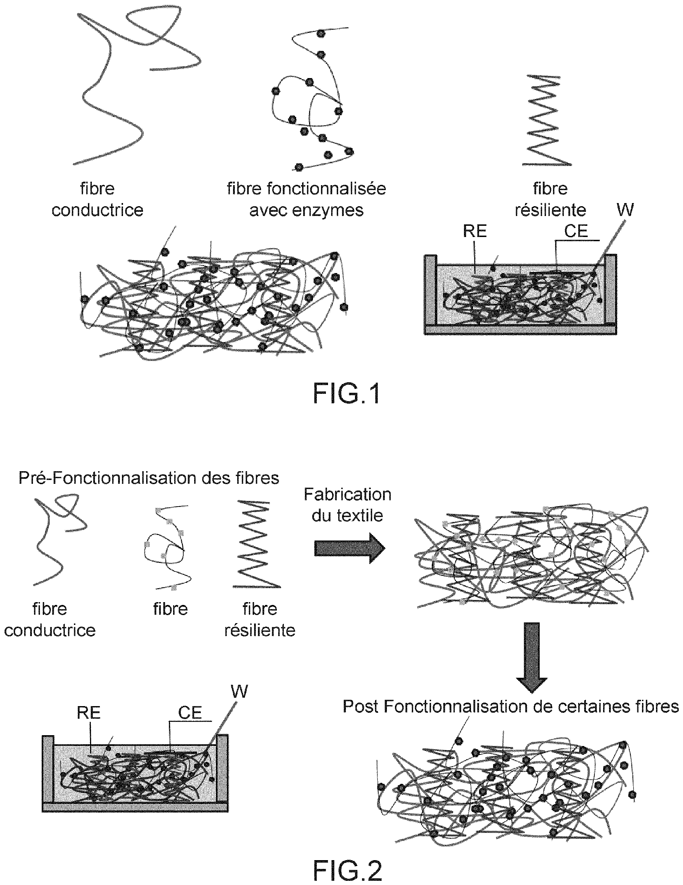

Dans ce procédé dont le principe est schématisé à la Figure 1, les fibres non-conductrices de l'électricité que sont les fibres en PET sont fonctionnalisées préalablement par des enzymes du type glucose oxydase.

Cette fonctionnalisation consiste tout d'abord, à soumettre les fibres à une oxydation de surface générant des fonctions polaires -OH ou-COOH à la surface des fibres puis les enzymes du type glucose oxydase sont chimiquement greffées sur les fibres via des liaisons peptidiques (ester activé et création de liaisons amides, estérification...).

Les fibres ainsi fonctionnalisées sont ensuite mélangées avec les fibres de carbone et les fibres résilientes LowMelt bicomposantes PET/coPET.

Afin de créer le matériau non tissé, la technique airlaid est utilisée. Pour ce faire, les fibres du mélange de fibres sont ouvertes puis insérées dans l'appareil où elles sont mélangées par courant d'air afin d'obtenir un non tissé ayant une répartition des fibres homogène. En sortie, une partie contrôlée du mélange de fibres est déposée par air sur un tapis formant ainsi le voile de masse surfacique entre 100 g/m² et 800 g/m², notamment entre 200 g/m² et 300 g/m².

Ce voile de masse est ensuite consolidé. La technique de consolidation utilisée est le liage thermique de préférence au four. Le matériau fibreux ainsi obtenu joue le rôle d'électrode de travail (WE) grâce aux fibres conductrices de l'électricité qu'il contient. A titre de contre-électrode (CE), on peut utiliser une grille de platine, un fil de platine, une plaque de titane platiné, une pâte de noir de carbone et de carbone activé encapsulés dans une grille d'inox ou une pâte de noir de carbone, de carbone activé et de téflon encapsulés dans une grille d'inox. A titre d'électrode de référence (RE), on peut utiliser une électrode au calomel saturé comme une électrode au calomel saturé en chlorure de potassium ou en chlorure de sodium, une électrode Ag/AgCI ou encore un fil de platine.

### III. Procédé de préparation de l'électrode selon la seconde variante.

Dans ce procédé dont le principe est schématisé à la Figure 2, les fibres en PET sont soumises à une oxydation de surface générant des fonctions polaires -OH ou -COOH à la surface des fibres puis les fibres en PET ainsi pré-fonctionnalisées sont mélangées avec les fibres de carbone et les fibres résilientes en LowMelt bicomposantes PET/coPET.

Le mélange ainsi obtenu est soumis à la technique airlaid comme décrit pour le procédé du point Il ci-dessus.

Une fois le voile de masse obtenu, il est mis en contact avec des enzymes du type glucose oxydase et soumis à des conditions permettant la fixation chimique de ces enzymes sur les fibres en PET pré-fonctionnalisées contenues dans le voile via des liaisons peptidiques (ester activé et création de liaisons amides, estérification...).

La suite du procédé à savoir la consolidation est identique à celle décrite pour le procédé du point Il ci-dessus.

### IV. Procédé de préparation de l'électrode selon la troisième variante.

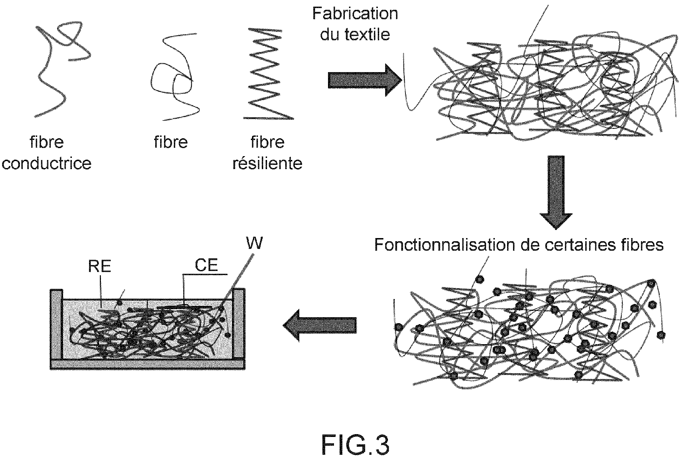

Dans cette troisième variante dont le principe est schématisé à la Figure 3, les fibres en PET, les fibres de carbone et les résilientes LowMelt bicomposantes PET/coPET sont mélangées toutes ensemble.

Le mélange ainsi obtenu est soumis à la technique airlaid comme décrit pour le procédé du point Il ci-dessus.

Une fois le voile de masse obtenu, il est mis en contact avec des enzymes du type glucose oxydase et soumis à des conditions permettant la fixation chimique de ces enzymes sur les fibres en PET contenues dans le voile via des liaisons covalentes.

La suite du procédé à savoir la consolidation est identique à celle décrite pour le procédé du point Il ci-dessus.

### RÉFÉRENCES

**[1]** Mahbur Rahman et al, 2010, "A comprehensive review of glucose biosensors based on nanostructured metal-oxyde", Sensor (Basel), vol. 10, pages 4855-4886.

## Revendications

1. Electrode pour biocapteur enzymatique se présentant sous forme d'un matériau fibreux et comprenant des fibres conductrices de l'électricité et des fibres non-conductrices de l'électricité,
tout ou partie desdites fibres non-conductrices de l'électricité étant fonctionnalisées par des enzymes, identiques ou différentes,
lesdites fibres conductrices de l'électricité n'étant pas fonctionnalisées par des enzymes,
l'électrode étant **caractérisée en ce que** lesdites enzymes sont réparties sur l'ensemble de la partie latérale des fibres non-conductrices de l'électricité.

2. Electrode selon la revendication 1, **caractérisée en ce qu'**elle comprend des fibres conductrices de l'électricité, un premier type de fibres non-conductrices de l'électricité, non fonctionnalisées par des enzymes et au moins un second type de fibres non-conductrices de l'électricité, différent du premier type de fibres non-conductrices de l'électricité, tout ou partie desquelles sont fonctionnalisées par des enzymes, identiques ou différentes.

3. Electrode selon la revendication 1 ou 2, **caractérisée en ce qu'**elle se présente sous forme d'un matériau fibreux, compressible et résilient.

4. Electrode selon l'une quelconque des revendications 1 à 3, **caractérisée en ce que** ledit matériau fibreux se présente sous forme d'un textile tissé, sous forme d'un textile tricoté ou sous forme d'un textile non tissé.

5. Electrode selon l'une quelconque des revendications 1 à 4, **caractérisée en ce que** lesdites fibres conductrices de l'électricité sont choisies dans le groupe constitué par les fibres métalliques, les fibres en carbone, les fibres en polymères ou copolymères conducteurs de l'électricité, les fibres en composites polymères chargés (CPC) et les fibres rendues conductrices via enduction ou métallisation.

6. Electrode selon l'une quelconque des revendications 1 à 5, **caractérisée en ce que** lesdites enzymes sont choisies dans le groupe constitué par les oxydoréductases, les oxygénases, les peroxydases, les catalases, les transhydrogénases, les déshydrogénases, les transférases, les hydrolases, les lyases et les ligases.

7. Electrode selon l'une quelconque des revendications 1 à 6, **caractérisée en ce que** ladite fonctionnalisation des fibres non-conductrices de l'électricité par des enzymes est directe ou indirecte.

8. Procédé de préparation d'une électrode pour biocapteur enzymatique selon l'une quelconque des revendications 1 à 7, lequel procédé comprend :
a1) la fonctionnalisation des fibres non-conductrices de l'électricité par des enzymes, sur l'ensemble de la partie latérale desdites fibres non-conductrices de l'électricité ;
b1) le mélange des fibres non-conductrices de l'électricité fonctionnalisées par des enzymes avec des fibres conductrices de l'électricité non fonctionnalisées par des enzymes et éventuellement des fibres non-conductrices de l'électricité ; et
c1) la mise en forme du mélange de fibres obtenues à l'étape b1) de façon à obtenir un matériau fibreux.

9. Procédé de préparation d'une électrode pour biocapteur enzymatique selon l'une quelconque des revendications 1 à 7, lequel procédé comprend :
a2) la fonctionnalisation des fibres non-conductrices de l'électricité par des fonctions réactives sur l'ensemble de la partie latérale desdites fibres non-conductrices de l'électricité ;
b2) le mélange des fibres non-conductrices de l'électricité fonctionnalisées par des fonctions réactives avec des fibres conductrices de l'électricité non fonctionnalisées par des enzymes et éventuellement des fibres non-conductrices de l'électricité ; et
c2) la mise en forme du mélange de fibres obtenues à l'étape b2) de façon à obtenir un matériau fibreux ;
la fonctionnalisation par des enzymes des fibres non-conductrices de l'électricité préalablement fonctionnalisées par des fonctions réactives étant réalisée soit après l'étape b2) et préalablement à l'étape c2), soit après l'étape c2).

10. Procédé de préparation d'une électrode pour biocapteur enzymatique selon l'une quelconque des revendications 1 à 7, lequel procédé comprend :
a3) le mélange de fibres conductrices de l'électricité non fonctionnalisées par des enzymes et de fibres non-conductrices de l'électricité, et
b3) la mise en forme du mélange de fibres obtenues à l'étape a3) de façon à obtenir un matériau fibreux,
la fonctionnalisation par des enzymes des fibres non-conductrices de l'électricité sur l'ensemble de la partie latérale desdites fibres non-conductrices de l'électricité étant réalisée soit après l'étape a3) et préalablement à l'étape b3), soit après l'étape b3).

11. Procédé selon l'une quelconque des revendications 8 à 10, **caractérisé en ce que**, lorsque le matériau fibreux est non tissé, la mise en forme lors desdites étapes c1), c2) et b3) est réalisée en utilisant une technique par voie sèche comme le procédé de cardage ou le procédé airlaid.

12. Procédé selon l'une quelconque des revendications 8 à 11, **caractérisé en ce que** le matériau fibreux obtenu suite auxdites étapes c1), c2) et b3) est soumis à une consolidation.

13. Biocapteur enzymatique à détection électrochimique, comprenant une électrode selon l'une quelconque des revendications 1 à 7.

## Patentansprüche

1. Elektrode für einen enzymatischen Biosensor, die als ein Fasermaterial vorliegt, und elektrisch leitende Fasern und elektrisch nicht-leitende Fasern umfassend,
wobei alle oder ein Teil der elektrisch nicht-leitenden Fasern durch Enzyme funktionalisiert sind, die gleich oder unterschiedlich sind,
wobei die elektrisch leitenden Fasern nicht durch Enzyme funktionalisiert sind,
wobei die Elektrode **dadurch gekennzeichnet ist, dass** die Enzyme über den gesamten lateralen Teil der elektrisch nicht-leitenden Fasern verteilt sind.

2. Elektrode nach Anspruch 1, **dadurch gekennzeichnet, dass** sie elektrisch leitende Fasern umfasst, einen ersten Typ von elektrisch nicht-leitenden Fasern, die nicht durch Enzyme funktionalisiert sind, und mindestens einen zweiten Typ von elektrisch nicht-leitenden Fasern, die sich vom ersten Typ von elektrisch nicht-leitenden Fasern unterscheiden, von denen alle oder ein Teil davon durch gleiche oder unterschiedliche Enzyme funktionalisiert sind.

3. Elektrode nach Anspruch 1 oder 2, **dadurch gekennzeichnet, dass** sie als Fasermaterial vorliegt, das komprimierbar und widerstandsfähig ist.

4. Elektrode nach einem der Ansprüche 1 bis 3, **dadurch gekennzeichnet, dass** das Fasermaterial als gewobene Textilie, als gestrickte Textilie oder als nichtgewobene Textilie vorliegt.

5. Elektrode nach einem der Ansprüche 1 bis 4, **dadurch gekennzeichnet, dass** die elektrisch leitenden Fasern aus der Gruppe ausgewählt sind, die aus metallischen Fasern, Kohlenstofffasern, Fasern aus elektrisch leitenden Polymeren oder Copolymeren, Fasern aus geladenen Polymerverbundwerkstoffen (CPC) und Fasern, die durch Beschichtung oder Metallisierung leitend gemacht werden, besteht.

6. Elektrode nach einem der Ansprüche 1 bis 5, **dadurch gekennzeichnet, dass** die Enzyme aus der Gruppe ausgewählt sind, die aus Oxidoreduktasen, Oxygenasen, Peroxidasen, Katalasen, Transhydrogenasen, Dehydrogenasen, Transferasen, Hydrolasen, Lyasen und Ligasen besteht.

7. Elektrode nach einem der Ansprüche 1 bis 6, **dadurch gekennzeichnet, dass** die Funktionalisierung der elektrisch nicht-leitenden Fasern durch Enzyme direkt oder indirekt ist.

8. Verfahren zur Herstellung einer Elektrode für einen enzymatischen Biosensor nach einem der Ansprüche 1 bis 7, wobei das Verfahren umfasst:
a1) die Funktionalisierung der elektrisch nicht-leitenden Fasern durch Enzyme über den gesamten lateralen Teil der elektrisch nicht-leitenden Fasern;
b1) die Mischung von elektrisch nicht-leitenden Fasern, die durch Enzyme funktionalisiert wurden, mit elektrisch leitenden Fasern, die nicht durch Enzyme funktionalisiert wurden, und gegebenenfalls elektrisch nicht-leitenden Faser; und
c1) die Formgebung der Mischung aus Fasern, die in Schritt b1) erhalten wurden, um ein Fasermaterial zu erhalten.

9. Verfahren zur Herstellung einer Elektrode für einen enzymatischen Biosensor nach einem der Ansprüche 1 bis 7, wobei das Verfahren umfasst:
a2) die Funktionalisierung der elektrisch nicht-leitenden Fasern durch reaktive Funktionen über den gesamten lateralen Teil der elektrisch nicht-leitenden Fasern;
b2) die Mischung von elektrisch nicht-leitenden Fasern, die durch reaktive Funktionen funktionalisiert wurden, mit elektrisch leitenden Fasern, die nicht durch Enzyme funktionalisiert wurden, und gegebenenfalls elektrisch nicht-leitenden Fasern; und
c2) die Formgebung der Mischung aus Fasern, die in Schritt b2) erhalten wurden, um ein Fasermaterial zu erhalten;
wobei die Funktionalisierung durch Enzyme von elektrisch nicht-leitenden Fasern, die zuvor durch reaktive Funktionen funktionalisiert wurden, entweder nach Schritt b2) und vor Schritt c2) realisiert wird oder nach Schritt c2).

10. Verfahren zur Herstellung einer Elektrode für einen enzymatischen Biosensor nach einem der Ansprüche 1 bis 7, wobei das Verfahren umfasst:
a3) die Mischung von elektrisch leitenden Fasern, die nicht durch Enzyme funktionalisiert wurden, und elektrisch nicht-leitenden Fasern, und
b3) die Formgebung der Mischung aus Fasern, die in Schritt a3) erhalten wurden, um ein Fasermaterial zu erhalten,
wobei die Funktionalisierung durch Enzyme von elektrisch nicht-leitenden Fasern über den gesamten lateralen Teil der elektrisch nicht-leitenden Fasern entweder nach Schritt a3) und vor Schritt b3) realisiert wird oder nach Schritt b3).

11. Verfahren nach einem der Ansprüche 8 bis 10, **dadurch gekennzeichnet, dass**, wenn das Fasermaterial nicht gewebt ist, die Formgebung in den Schritten c1), c2) und b3) unter Verwendung einer Trockenmethode wie dem Kardierungsverfahren oder dem Airlaid-Verfahren realisiert wird.

12. Verfahren nach einem der Ansprüche 8 bis 11, **dadurch gekennzeichnet, dass** das nach den Schritten c1), c2) und b3) erhaltene Fasermaterial einer Konsolidierung unterzogen wird.

13. Enzymatischer Biosensor mit elektrochemischer Detektion, eine Elektrode nach einem der Ansprüche 1 bis 7 umfassend.

## Claims

1. Electrode for enzymatic biosensor being in the form of a fibrous material and comprising electrically conductive fibres and electrically non-conductive fibres,
all or part of said non-conductive fibres being functionalised by enzymes, identical or different,
said electrically conductive fibres being non-functionalised by enzymes, and
said enzymes being distributed over the whole of the lateral part of said non-conductive fibres.

2. Electrode according to claim 1, **characterized in that** the electrode comprises electrically conductive fibres, a first type of electrically non-conductive fibres, non-functionalised by enzymes and at least one second type of electrically non-conductive fibres, different from the first type of electrically non-conductive fibres, all or part of which are functionalised by enzymes, identical or different.

3. Electrode according to claim 1 or 2, **characterized in that** the electrode is in the form of a fibrous material, compressible and resilient.

4. Electrode according to any of claims 1 to 3, **characterized in that** said fibrous material is in the form of a woven textile, in the form of a knitted textile or in the form of a non-woven textile.

5. Electrode according to any of claims 1 to 4, **characterized in that** said electrically conductive fibres are chosen from the group consisting of metal fibres, carbon fibres, fibres made of electrically conductive polymers or copolymers, fibres made of conductive polymer composites (CPC) and fibres made to be conductive via coating or metallisation.

6. Electrode according to any of claims 1 to 5, **characterized in that** said enzymes are chosen from the group consisting of oxidoreductases, oxygenases, peroxidases, catalases, transhydrogenases, dehydrogenases, transferases, hydrolases, lyases and ligases.

7. Electrode according to any of claims 1 to 6, **characterized in that** said functionalisation of the electrically non-conductive fibres by enzymes is direct or indirect.

8. Method for preparing an electrode for enzymatic biosensor according any of claims 1 to 7, which method comprises:
a1) functionalisation of the electrically non-conductive fibres by enzymes on the whole of the lateral part of said electrically non-conductive fibres;
b1) mixing of the electrically non-conductive fibres functionalised by enzymes with electrically conductive fibres non-functionalised by enzymes and optionally electrically non-conductive fibres; and
c1) shaping of the mixture of fibres obtained at step b1) so as to obtain a fibrous material.

9. Method for preparing an electrode for enzymatic biosensor according to any of claims 1 to 7, which method comprises:
a2) functionalisation of the electrically non-conductive fibres by reactive functions on the whole of the lateral part of said electrically non-conductive fibres;
b2) mixing of the electrically non-conductive fibres functionalised by reactive functions with electrically conductive fibres non-functionalised by enzymes and optionally electrically non-conductive fibres; and
c2) shaping of the mixture of fibres obtained at step b2) so as to obtain a fibrous material;
the functionalisation by enzymes of the electrically non-conductive fibres functionalised beforehand by reactive functions being carried out either after step b2) and prior to step c2), or after step c2).

10. Method for preparing an electrode for enzymatic biosensor according to any of claims 1 to 7, which method comprises:
a3) mixing of the electrically conductive fibres non-functionalised by enzymes and electrically non-conductive fibres, and
b3) shaping of the mixture of fibres obtained at step a3) so as to obtain a fibrous material,
the functionalisation by enzymes of the electrically non-conductive fibres on the whole of the lateral part of said electrically non-conductive fibres being carried out either after step a3) and prior to step b3), or after step b3).

11. Method according to any of claims 8 to 10, **characterized in that** when the fibrous material is non-woven, the shaping during said steps c1), c2) and b3) is carried out using a dry technique such as the carding or the airlaid process.

12. Method according to any of claims 8 to 11, **characterized in that** the fibrous material obtained following said step c1), c2) and b3) is subjected to a consolidation.

13. Electrochemical detection enzymatic biosensor, comprising an electrode according to any of claims 1 to 7.
